(19) Europäisches Patentamt
European Patent Office
Office européen des brevets

(11) Publication number: **0 198 605 A1**

# (12) EUROPEAN PATENT APPLICATION

(21) Application number: **86301960.0**

(22) Date of filing: **18.03.86**

(51) Int. Cl.⁴: **C 07 D 277/22**, A 61 K 31/425

(30) Priority: **19.03.85 JP 53256/85**

(43) Date of publication of application: **22.10.86 Bulletin 86/43**

(84) Designated Contracting States: **CH DE FR GB IT LI NL SE**

(71) Applicant: **TEIJIN LIMITED, 11 Minamihonmachi 1-chome Higashi-ku, Osaka-shi Osaka 541 (JP)**

(72) Inventor: **Yamamoto, Itaru, 1-102, Hanajiri Kikyo-machi, Okayama-shi Okayama (JP)**
Inventor: **Koyama, Yasushi, 1602, Green Mountain Drive, 248 G Little Rock, AR 72211 (US)**
Inventor: **Komoriya, Keiji, 1532-117, Uchikoshi-machi, Hachioji-shi Tokyo (JP)**
Inventor: **Hayami, Masaaki, 2-22-17, Fukutomi Nishi, Okayama-shi Okayama (JP)**
Inventor: **Okazaki, Funeki, 348-14, Toushinden, Okayama-shi Okayama (JP)**

(74) Representative: **Votier, Sidney David et al, CARPMAELS & RANSFORD 43, Bloomsbury Square, London WC1A 2RA (GB)**

## (54) Prophylaxis of pharmacotherapy of liver disorders.

(57) Methincyanine compounds having the following general formula (I) are effective for the prophylaxis or pharmacotherapy of liver disorders or hepatic diseases of warm-blooded animals.

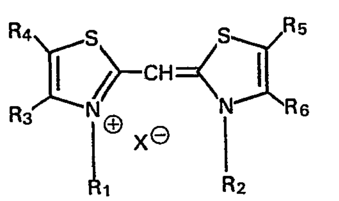

(I)

wherein $R_1$ and $R_2$ may be the same or different and represent an unsubsituted or substituted alkyl group, $R_3$, $R_4$, $R_5$, and $R_6$ independently represent a hydrogen atom or an alkyl group, and $X^{\ominus}$ represents an anion.

EP 0 198 605 A1

# PROPHYLAXIS OR PHARMACOTHERAPY OF LIVER DISORDERS

## BACKGROUND OF THE INVENTION

### 1. Field of the Invention

The present invention relates to the prophylaxis or pharmacotherapy of liver disorders or hepatic diseases. More specifically, it relates to the prophylaxis or pharmacotherapy of liver disorders of warm-blooded animals by the administration of methincyanine compounds, use of the methincyanine compounds for the production of pharmaceutical preparations for the prophylaxis or pharmacotherapy of liver disorders, and certain novel methincyanine compounds. According to the present invention, an excellent prophylaxis or treatment of liver disorders such as toxic hepatopathy, jecur adiposum, acute hepatitis, chronic hepatitis, and hepatocirrhosis is obtained.

### 2. Description of the Prior Art

Certain methincyanine compounds such as pionin (i.e., 2-[2-(3-heptyl-4-methyl-4-thiazolin-2-ylidene)-methyl]-3-heptyl thiazolinum iodide) and platonin (i.e., 2,2'-[3-[2-(3-heptyl-4-methyl-4-thiazolin-2-ylidene)-ethylidene]propenylene]bis[3-heptyl-4-methyl thiazolinum iodide]) have been used as a photosensitive dye in a photosensitive film. Furthermore, recently, the pharmaceutical activities of these photosensitive dye compounds have been noted, and they are now used in various cosmetic products and non-medical products.

For example, pionin, platonin, and similar compounds are formulated into, for example, cosmetic products, due to their excellent antimicrobial properties as disclosed in Pharmacia, Vol. 20, No. 11, 1123 (1984). It is also disclosed in, for example, Japanese Unexamined Patent Publication (Kokai) Nos. 54-151133 and 55-69513 that methincyanine compounds such as pionin are effective as an antineoplastic agent due to their strong antineoplastic activities. Furthermore, it is reported in, for

example, U.S. Patent No. 3562261 that the nicotinates of methincyanine compounds such as nicotinates of pionin are effective as medicaments for promoting the metabolism of the cellular structure, the growth of hair, and the regeneration of skin tissue, wounds, and the like by external application.

SUMMARY OF THE INVENTION

An object of the present invention is to develop novel pharmacological activities of methincyanine compounds such as pionin. In this connection, it has been found that the methincyanine compounds have pharmacological activities such that they strongly inhibit liver necrocytosis induced by carbon tetrachloride and liver disorders induced by D-galactosamine and, therefore, the methincyanine compounds are extremely effective for the prophylaxis or pharmacotherapy of liver disorders or hepatic diseases.

Accordingly, a further object of the present invention is to provide a prophylaxis or pharmacotherapy of liver disorders or hepatic diseases by the administration of methincyanine compounds.

Another object of the present invention is the use of methincyanine compounds for the production of pharmacological preparations for the prophylaxis or pharmacotherapy of liver disorders or hepatic diseases.

A still further object of the present invention is to provide novel methincyanine compounds hitherto not reported or disclosed in any literature, which are remarkably effective for the prophylaxis or pharmacotherapy of liver disorders or hepatic diseases.

A still further object of the present invention is to provide pharmacological preparations suitable for use in the prophylaxis or pharmcotherapy of liver disorders or hepatic diseases containing as an active component the novel methincyanine compounds.

Other objects and advantages of the present invention will be apparent from the following description.

According to the present invention, there is provided a prophylaxis or pharmacotherapy of liver disorders or hepatic diseases for warm-blooded animals by administrating thereto an effective amount of a methincyanine compound having the general formula [I].

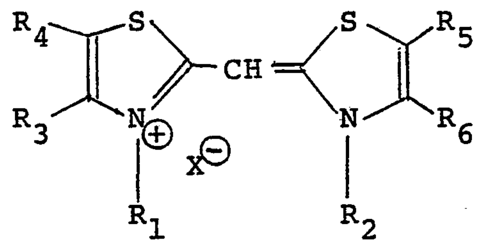

... [I]

wherein $R_1$ and $R_2$ may be the same or different and represent an unsubstituted or substituted alkyl group, $R_3$ , $R_4$ , $R_5$ , and $R_6$ independently represent a hydrogen atom or an alkyl group, and $X^{\ominus}$ represents an anion.

DESCRIPTION OF THE PREFERRED EMBODIMENTS

In the above-mentioned general formula (I), $R_1$ and $R_2$ are the same or different and represent alkyl groups which may or may not have a substitutent. The alkyl groups are linear or branched alkyl groups having 1 to 20 carbon atoms. Examples of such alkyl groups are methyl, ethyl, propyl, isopropyl, butyl, isobutyl, pentyl, hexyl, heptyl, octyl, nonyl, decyl, undecyl, tridecyl, tetradecyl, hexadecyl, nonadecyl, and eicosyl groups. The substituents which may be substituted in the alkyl groups include, for example, a hydroxyl group.

In the above-mentioned general formula (I), $R^3$, $R^4$, $R^5$, and $R^6$ are a hydrogen atom and alkyl groups. Examples of such alkyl groups are linear or branched alkyl groups having 1 to 8 carbon atoms, such as methyl, ethyl, propyl, isopropyl, butyl, isobutyl, pentyl, hexyl, and octyl groups. In the general formula (I), X represents an anion. Examples of such anions are halogen anions such as an iodo ion, bromo ion, and chloro ion; inorganic acid anions such as a phosphate ion, nitrate ion, sulfate ion, and perchlorate ion; and organic acid anions such as a thiocyanate ion, acetate

ion, p-toluenesulfonate ion, orotate ion, and nicotinate ion.

The above-mentioned methincyanine compounds having the same alkyl or substituted alkyl groups as $R_1$ and $R_2$ groups are known compounds, which can be produced by known methods disclosed in, for example, The Chemistry of Synthetic Dyes, Vol. 2, 1155, Academic Press (1952) and The Theory of the Photographic Process, 3rd edition, N.Y., The Macmillan Company, 206.

On the other hand, the methincyanine compounds having the general formula (I) in which the $R_1$ and $R_2$ are the different alkyl or substituted alkyl groups, are novel compounds not hitherto reported or disclosed in any literature. These novel methincyanine compounds are represented by the following general formula (II):

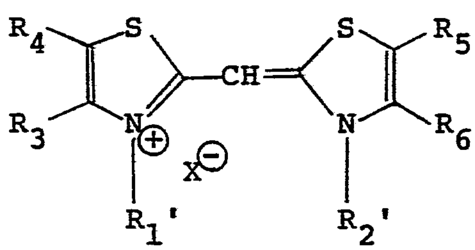

... [II]

wherein $R_1$' and $R_2$' are different from each other and represent an unsubstituted or substituted alkyl group, $R_3$ , $R_4$ , $R_5$ , and $R_6$ independently represent a hydrogen atom or an alkyl group, and $X^{\ominus}$ represents an anion.

In the above-mentioned general formula (II), $R_1$ and $R_2$ are different from each other and represent alkyl groups which may or may not have a substitutent. The alkyl groups are linear or branched alkyl groups having 1 to 20 carbon atoms. Examples of such alkyl groups are methyl, ethyl, propyl, isopropyl, butyl, isobutyl, pentyl, hexyl, heptyl, octyl, nonyl, decyl, undecyl, tridecyl, tetradecyl, hexadecyl, nonadecyl, and eicosyl groups. The substituents which may be substituted in the alkyl groups include, for example, a hydroxyl group.

In the above-mentioned general formula (II), $R^3$, $R^4$, $R^5$, and $R^6$ are a hydrogen atom and alkyl groups, and X represents an anion. Typical examples of the $R_3$, $R_4$, $R_5$, and $R_6$ groups and X are the same as defined in the general formula (I).

These novel methincyanine compounds having the general formula (II) can be similarly produced by using the above-mentioned known methods. For example, the compound (II) can be produced as follows:

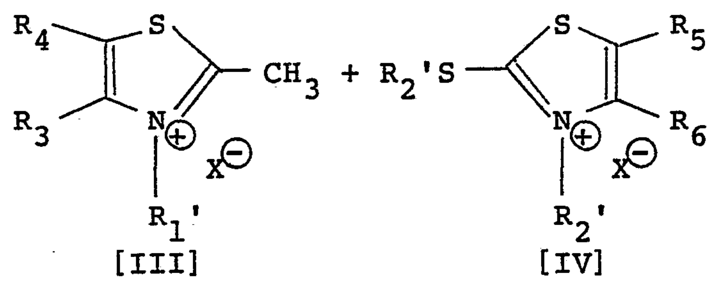

[II]

[III] [IV]

wherein $R_1'$, $R_2'$, $R^3$, $R^4$, $R^5$, and $R^6$, and X are as defined above.

The compounds (III) and (IV) can be produced in a known method disclosed in, for example, The Cyanine Dyes and Related Compounds, New York, John Willey and Sons (1964).

The reaction of the compound having the general formula (III) with the compound having the general formula (IV) can be carried out, for example, in the presence of an organic base in an organic solvent at an elevated temperature. Examples of the organic bases usable in the reaction are organic amines such as trimethylamine, triethylamine, pyridine, and piperidine. Examples of the organic solvents are alcohols such as methanol, ethanol, propanol, and butanol; acetone; dimethylformamide; and dimethylsulfoxide.

The compound (III) and the compound (IV) are preferably used in an equal mole amount or similar amount in the reaction. The organic base is preferably used in an amount of 1 to 3 moles based on 1 mole of the compound (III) or (IV). The reaction is preferably carried out at a temperature of 60°C to 70°C for 1 to

6 hours. The recovery and purification of the desired reaction product can be carried out by any conventional method, e.g., separation, washing, and recrystallization.

Typical examples of the methincyanine compounds having the general formula (I) are as follows:

A. Compounds having the same unsubstituted or substituted alkyl groups in $R_1$ and $R_2$ of the general formula (I)

1) 2-[2-(3-ethyl-4-methyl-4-thiazolin-2-ylidene) methyl]-3-ethyl-4-methyl thiazolinium iodide,

2) 2-[2-(3-propyl-4-methyl-4-thiazolin-2-ylidene) methyl]-3-propyl-4-methyl thiazolinium iodide,

3) 2-[2-(3-heptyl-4-methyl-4-thiazolin-2-ylidene) methyl]-3-heptyl-4-methyl thiazolinium iodide,

4) 2-[2-(3-heptyl-4-methyl-4-thiazolin-2-ylidene) methyl]-3-heptyl-4-methyl thiazolinium nitrate,

5) 2-[2-(3-heptyl-4-methyl-4-thiazolin-2-ylidene) methyl]-3-heptyl-4-methyl thiazolinium acetate,

6) 2-[2-(3-heptyl-4-methyl-4-thiazolin-2-ylidene) methyl]-3-heptyl-4-methyl thiazolinium orotate,

7) 2-[2-(3-heptyl-4-methyl-4-thiazolin-2-ylidene) methyl]-3-heptyl-4-methyl thiazolinium nicotinate,

8) 2-[2-(3-pentyl-4-methyl-4-thiazolin-2-ylidene) methyl]-3-pentyl-4-methyl thiazolinium iodide,

9) 2-[2-(3-octyl-4-methyl-4-thiazolin-2-ylidene) methyl]-3-octyl-4-methyl thiazolinium iodide,

B. Compounds having the different unsaturated or saturated alkyl groups in $R_1$ and $R_2$ of the general formula (I) (i.e., Compounds having the general formula (II))

1) 2-[2-(3-dodecyl-4-methyl-4-thiazolin-2-ylidene) methyl]-3-ethyl-4-methyl thiazolinium iodide,

2) 2-[2-(3-(2-hydroxyethyl)-4-methyl-4-thiazolin-2-ylidene) methyl]-3-ethyl-4-methyl thiazolinium chloride,

3) 2-[2-(3-heptyl-4-methyl-4-thiazolin-2-ylidene) methyl]-3-butyl-4-methyl thiazolinium iodide,

4) 2-[2-(3-heptyl-4-methyl-4-thiazolin-2-ylidene) methyl]-3-butyl-4-methyl thiazolinium chloride,

5) 2-[2-(3-heptyl-4-methyl-4-thiazolin-2-ylidene) methyl]-3-butyl-4-methyl thiazolinium nicotinate,

6) 2-[2-(3-dodecyl-4-methyl-4-thiazolin-2-ylidene) methyl]-3-butyl-4-methyl thiazolinium iodide,

7) 2-[2-(3-dodecyl-4-methyl-4-thiazolin-2-ylidene) methyl]-3-butyl-4-methyl thiazolinium chloride,

8) 2-[2-(3-octadecyl-4-methyl-4-thiazolin-2-ylidene) methyl]-3-butyl-4-methyl thiazolinium iodide,

9) 2-[2-(3-2-hydroxyethyl-4-methyl-4-thiazolin-2-ylidene) methyl]-3-butyl-4-methyl thiazolinium chloride,

10) 2-[2-(3-methyl-4-methyl-4-thiazolin-2-ylidene) methyl]-3-heptyl-4-methyl thiazolinium iodide,

11) 2-[2-(3-(2-hydroxyethyl)-4-methyl-4-thiazolin-2-ylidene) methyl]-3-heptyl-4-methyl thiazolinium chloride,

12) 2-[2-(3-decyl-4-methyl-4-thiazolin-2-ylidene) methyl]-3-butyl-4-methyl thiazolinium iodide,

13) 2-[2-(3-decyl-4-methyl-4-thiazolin-2-ylidene) methyl]-3-butyl-4-methyl azolinium chloride,

14) 2-[2-(3-nonyl-4-methyl-4-thiazolin-2-ylidene) methyl]-3-pentyl-4-methyl thiazolinium iodide,

15) 2-[2-(3-nonyl-4-methyl-4-thiazolin-2-ylidene) methyl]-3-pentyl-4-methyl thiazolinium chloride,

16) 2-[2-(3-butyl-4-methyl-4-thiazolin-2-ylidene) methyl]-3-heptyl-5-methyl thiazolinium iodide,

17) 2-[2-(3-butyl-4-methyl-4-thiazolin-2-ylidene) methyl]-3-heptyl-4,5-dimethyl thiazolinium iodide,

18) 2-[2-(3-butyl-4-methyl-4-thiazolin-2-ylidene methyl]-3-methyl-4-hexyl-5-methyl thiazolinium perchlorate,

19) 2-[2-(3-butyl-4-methyl-4-thiazolin-2-ylidene) methyl]-3-decyl-5-methyl thiazolinium iodide,

According to a study by the inventors, the methincyanine compounds having the general formula (I) can strongly inhibit liver necrocytosis induced by carbon tetrachloride and also strongly suppress liver disorders induced by D-galactosamine. Accordingly, these methincyanine compounds are effective for the prophylaxis or pharmacotherapy of acute or chronic liver disorders or hepatic diseases, of warm-blooded animals, such as toxic hepatopathy, jecur adiposum, acute hepatitis, chronic hepatitis, and hepatocirrhosis. Especially, as mentioned above, the methincyanine compounds having the general formula (II) are novel compounds hitherto not reported or disclosed in any literature. Furthermore, the above-mentioned pharmaceutical activities, i.e., the inhibition or surpression activities of liver disorders or hepatic diseases of the methincyanine compounds, are completely different from the known activities thereof (i.e., the antimicrobial activities and antineoplastic activities).

When the methincyanine compounds according to the present invention are used for the prophylaxis or pharmacotherapy of liver disorders or hepatic diseases of warm-blooded animals, the compounds are preferably administered thereto in a dosage of 50 μg to 5 mg/head/day, although the dosage depends upon the symptoms. The administration can be orally or parenterally carried out one to four times a day.

The orally applicable preparations of the present invention can be in the form of, for example, tablets, pills, granules, powders, liquids, suspensions, and capsules.

When produced in tablet form, the methincyanine compounds can be formed in any conventional manner by using conventional ingredients. Examples of such ingredients are excipients such as lactose, starch, and crystalline celluloses; binders such as carboxymethyl

cellulose, methyl cellulose, and polyvinyl pyrrolidone; disintegration agents such as sodium alginate and sodium bicarbonate; and the like.

The pharmaceutical preparations of the present invention in the form of liquids and suspensions can be produced in any conventional manner by using, for example, glycerol esters such as tricapryrin and triacetin and alcohols such as ethanol. The pharmaceutical preparations in the form of capsules can be produced by filling the granules, powder, or liquids into capsules.

The pharmaceutical preparations of the present invention can be used for injections, such as endovenous, intramuscular, or hypodermic injection. In the production of these preparations, various solvents such as physiological saline, ethanol, and propylene glycol and optional preservatives and stabilizers can be used as usual. For rectal administration, the present pharmaceutical preparations can be in the form of a conventional suppository using gelatin soft capsules. For percutaneous administration, the present pharmaceutical preparations can be formed as ointments. These forms of the preparations can be produced in any conventional manner.

The methincyanine compounds according to the present invention have a low toxicity and high stability. For example, the $LD_{50}$ of pionin (i.e., 2-[2-(3-heptyl-4-methyl-4-thiazolin-2-ylidene) methyl]-3-heptyl-4-methyl thiazolinium iodide) is 460 mg/kg (oral in the case of male rats) and 503 mg/kg (oral in the case of female rats) as disclosed in Nihon Koshohin Kagaku Kaishi, Vol. 8, No. 1, 1984.

EXAMPLE

The present invention will now be described in detail with reference to, but is by no means limited to, the following examples.

Example 1

Synthesis of 2-[2-(3-dodecyl-4-methyl-4-thiazolin-2-ylidene) methyl]-3-butyl-4-methyl thiazolinium iodide

(i) A 1 mol amount of 2-mercapto-4-methylthiazol and 2.5 mol of butyl p-toluenesulfonate were charged into an eggplant type flask and were then heated at a temperature of 130°C to 140°C for 8 hours on an oil bath. After the reaction, the resultant viscous fluid was cooled and then washed with ether several times. Thus, viscous 2-butylthio-3-butyl-4-methyl thiazolinium p-toluene sulfonate was obtained.

(ii) A 1 mol amount of 2,4-dimethyl-4-thiazol and 1.1 mol of dodecyl p-chlorobenzenesulfonate were charged into an eggplant type flask and were then heated at a temperature of 130°C to 140°C for 8 hours on an oil bath. After the reaction, the resultant viscous fluid was cooled and then washed several times with ether and a mixture of ether and acetone to form crystals. The crystals thus obtained were recovered by filtration and then washed several times with a mixture of ether and acetone. The products were recrystallized from a mixed solvent of ethanol and acetone (1:3) to obtain 2,4-dimethyl-3-N-dodecyl thiazolinium p-chlorobenzene sulfonate.

(iii) A 14.3 g amount of the 2-butylthio-3-butyl-4-methyl thiazlinium p-toluene sulfonate obtained in (i) above and 10 g of the 2,4-dimethyl-3-dodecyl thiazolinium p-chlorobenzenesulfonate obtained in (ii) above were dissolved in 60 ml of ethanol. To the resultant solution, 20 ml of triethylamine was added and the mixture was allowed to react at a temperature of 60°C for 6 hours while stirring. The solvent was distilled from the reaction mixture in vacuo. To the residue, 30 ml of methanol and, subsequently, 20 g of potassium iodide were added. The mixture was heated for one hour on a water bath and was then cooled to form yellow crystals. The crystals were recovered by filtration and thoroughly washed with water and, subsequently, with a

mixture of ethanol and ether. Thus, the desired compound having the following properties was obtained.

m.p.: 224 - 225°C

$\lambda_{max}$ (nm): 410

IR (KBr) $\nu_{max}$: 3050, 2920, 2850, 1520, 1380, 1280

Example 2

Synthesis of 2-[2-(3-dodecyl-4-methyl-4-thiazolin-2-ylidene) methyl]-3-butyl-4-methyl thiazolinium chloride

The desired compound was prepared by treating the compound obtained in Example 1 with silver chloride. The properties of the resultant compound are as follows:

m.p.: 203 - 204°C

$\lambda_{max}$ (nm): 411

IR (KBr) $\nu_{max}$: 3100, 2920, 2850, 1530, 1380, 1350, 1280

Example 3

Synthesis of 2-[2-(3-dodecyl-4-methyl-4-thiazolin-2-ylidene) methyl]-3-ethyl-4-methyl thiazolinium iodide

The desired compound was prepared from 2-ethylthio-3-ethyl-4-methyl thiazolinium iodide and 2,4-dimethyl-3-dodecyl thiazolinium p-chlorobenzene sulfonate in the same manner as in the procedure (iii) of Example 1.

The properties of the resultant compound are as follows:

m.p.: 206 - 207°C

$\lambda_{max}$ (nm): 410

IR (KBr) $\nu_{max}$: 3050, 2910, 2850, 1520, 1430, 1380, 1340, 1170

Example 4

Synthesis of 2-[2-(3-butyl-4-methyl-4-thiazolin-2-ylidene) methyl]-3-heptyl-5-methyl thiazolinium iodide

The desired compound was prepared from 2,5-dimethyl-3-heptyl thiazolinium iodide and 2-butylthio-

3-butyl-4-methyl thiazolinium p-toluenesulfonate in the same manner as in the procedure (iii) of Example 1. The properties of the resultant compound are as follows:

m.p.: 189 - 193°C

$\lambda_{max}$ (nm): 413

IR (KBr) $\nu_{max}$: 3070, 2930, 2850, 1530, 1290, 1210, 860

Example 5

Synthesis of 2-[2-(3-butyl-4-methyl-4-thiazolin-2-ylidene) methyl]-3-heptyl-4,5-dimethyl thiazolinium iodide

The desired compound was prepared from 2,4,5-trimethyl-3-heptyl thiazolinium iodide and 2-butylthio-3-butyl-4-methyl thiazolinium p-toluenesulfonate in the same manner as in the procedure of Example 1. The properties of the resultant compound are as follows:

m.p.: 199 - 201°C

$\lambda_{max}$ (nm): 417

IR (KBr) $\nu_{max}$: 3070, 2910, 2850, 1520, 1370, 1260, 1210

Example 6

Synthesis of 2-[2-(3-butyl-4-methyl-4-thiazolin-2-ylidene) methyl]-3-methyl-4-hexyl-5-methyl thiazolinium perchlorate

The desired compound was prepared from 2,5-dimethyl-4-hexyl-3-methylthiazolinium iodide and 2-butylthio-3-butyl-4-methyl thiazolinium p-toluenesulfonate in the same manner as in the procedure (iii) of Example 1, followed by treating the resultant iodide with sodium perchlorate. The properties of the resultant compound are as follows:

m.p.: 136 - 137°C

$\lambda_{max}$ (nm): 416

IR (KBr) $\nu_{max}$: 3120, 2960, 2940, 2850, 1560, 1280, 1090, 860

Example 7

Synthesis of 2-[2-(3-butyl-4-methyl-4-thiazolin-2-ylidene) methyl]-3-decyl-5-methyl thiazolinium iodide

The desired compound was prepared from 2,5-dimethyl-3-decylthiazolinium iodide and 2-butylthio-3-butyl-4-methyl thiazolinium p-toluenesulfonate in the same manner as in the procedure (iii) of Example 1. The properties of the resultant compound are as follows:

m.p.: 180 - 181°C

$\lambda_{max}$ (nm): 413

IR (KBr) $\nu_{max}$: 2810, 2650, 1520, 1360, 1280, 1200

Example 8

Synthesis of 2-[2-(3-decyl-4-methyl-4-thiazolin-2-ylidene) methyl]-3-butyl-4-methylthiazolinium iodide

The desired compound was prepared from 2,4-dimethyl-3-decylthiazolinium iodide and 2-butylthio-3-butyl-4-methylthiazolinium p-toluenesulfonate in the same manner as in the procedure (iii) of Example 1. The properties of the resultant compound are as follows:

m.p.: 225 - 226°C

$\lambda_{max}$ (nm): 413

Example 9

Synthesis of 2-[2-(3-(2-hydroxyethyl)-4-methyl-4-thiazolin-2-ylidene) methyl]-3-butyl-4-methylthiazolinium chloride

The desired compound was prepared from 2,4-dimethyl-3-(2-hydroxyethyl) thiazolinium iodide and 2-butylthio-3-butyl-4-methylthiazolinium p-toluenesulfonate in the same manner as in the procedure (iii) of Example 2. The properties of the resultant compound are as follows:

m.p.: 222°C

$\lambda_{max}$ (nm): 412

Example 10

Effect of methincyanine compound on hepatic necrocytosis induced by carbon tetrachloride (in vitro)

Liver cells were prepared according to a method disclosed in Nakamura et al "Proteins, Nucleic Acids, and Enzymes" Vol. 24, pages 55-76 (1981). That is, the rat liver cells, which were previously peritoneally treated with phenobarbital at 80 mg/kg/day for 3 days, were collected by centrifugal separation after being subjected to 0.05% collagenase perfusion.

The liver cells thus obtained were suspended in a William's E culture medium containing 10% fetal calf serum, dexamethasone ($10^{-6}$ M) and insulin (0.02 unit/ml). A 3 ml amount of the cell dispersion having a cell content of 5 x $10^5$ cells/ml was placed in a 25 mm Falcon dish and was then incubated at a temperature of 37°C under an atmosphere of 5% $CO_2$ and 95% air for 3 hours. After 3 hours incubation, the number of surviving liver cells in the incubated dish was measured according to a trypan blue exclusion test. The average value thereof was used as a control value for the survival percentage of the liver cells in the pre-incubation.

Then, a culture medium containing 2-[2-(3-heptyl-4-methyl-4-thiazolin-2-ylidene) methyl]-3-heptyl thiazolinium iodide (i.e., pionin) dissolved in ethanol was substituted after removing non-adhesive cells and was then incubated for a further one hour. The concentrations of pionin in the culture medium were 0.94 x $10^{-6}$, 1.8 x $10^{-7}$, 1 x $10^{-8}$, 1 x $10^{-9}$, and 1 x $10^{-10}$ mole and, as a control or comparative test, the culture medium itself, the culture medium containing 0.5% of ethanol, and the culture medium containing 0.25 μm/ml of carbon tetrachloride were used.

Then, 0.25 μm/ml of carbon tetrachloride was added to each culture dish and the incubation was carried out for a further 3 hours under the same conditions mentioned above. No further carbon tetrachloride was added to the comparative culture containing carbon tetrachloride. After 3 hours incubation, the number of survival liver cells was measured according to a trypan blue exclusion

test. The percentage ratio of the mean survival number to the survival number of the above-mentioned pre-incubation was then calculated.

The results are shown in Table 1, in which the mean survival cell percentage (%) and standard deviation thereof in the culture dish containing 4 to 29 liver cells (i.e., N). Statistical significance was evaluated by Student's t-test.

Table 1

| No. | Sample | Survival ratio (%) | N |
|---|---|---|---|
| 1 | Control | $87.2 \pm 17.3^{*2}$ | 16 |
| 2 | Ethanol | $81.6 \pm 18.3^{*2}$ | 16 |
| 3 | $CCl_4$ | $34.2 \pm 12.1$ | 29 |
| 4 | $CCl_4$ + Pionin ($1 \times 10^{-10}$ M) | $63.2 \pm 23.0^{*2}$ | 4 |
| 5 | " + " ($1 \times 10^{-9}$ M) | $63.4 \pm 6.8^{*2}$ | 4 |
| 6 | " + " ($1 \times 10^{-8}$ M) | $63.5 \pm 19.0^{*2}$ | 4 |
| 7 | " + " ($1.8 \times 10^{-7}$ M) | $47.1 \pm 8.1^{*2}$ | 12 |
| 8 | " + " ($0.94 \times 10^{-6}$ M) | $43.6 \pm 11.6^{*1}$ | 12 |

*1: The difference between test Nos. 3 and 8 is regarded as significant with a level of significance of 1%

*2: The differences between these tests and test No. 3 are regarded as significant with a level of significance of 5%

As is clear from the results of Table 1, pionin has an activity capable of inhibiting liver necrocytosis.

Example 11

Effect of methincyanine compound on hepatic disorders induced by D-galactosamine

In the evaluation tests, 8 to 9 weeks old Wister male rats were used. Five rats were used in each test

group. The rats were given standard chow and water *ad libitum*.

D-Galactosamine hydrochloride (GalN) was dissolved in sterilized physiological saline and the pH thereof was adjusted to 7. The resultant solution was peritoneally administered to each rat at a dosage of 250 mg/kg. To one control group, only GalN was injected, and physiological saline was administered to the other control group instead of pionin. Furthermore, only physiological saline but no GalN was injected to the physiological saline administration group.

The pionin was administered as follows. It is diluted 10, 100, and 1000 times with dextrose and these diluted powders were dissolved in saline. All drugs were intravenously or orally administered at doses of 0.1, 0.5, 1, and 10 μg/kg. The administration was carried out from five days before to the day after the GalN injection.

The rats were killed two days after the injection of GalN and blood was collected. The blood samples were allowed to stand at room temprature for one hour and serum was then centrifugally separated at 2500 rpm for 10 minutes.

The determination of GOT and GPT in the serum samples was carried out according to a modification of the Reitman-Framkel method [see Amir. J. Clin. Pothil. 28, 56 (1957) and J. Clin. Invest. 34, 131 (1955)], the determination of bilirubin was carried out according to a modification of Jendrassik-Grof method [see Clinical Chemistry, 19, 984-993 (1973)], and the determination of the complement value was carried out according to the Mayer method (see Experimental Immunochemistry, 2, 133-240, 1961).

The results are shown in Table 2 (i.e., intravenous administration of pionin) and Table 3 (i.e., oral administration of pionin). Disodium cromoglycate (i.e., DSCG) was used as a control.

Table 2

| No. | GalN (i.p.) | Enzyme activity | | Bilirubin (mg/dl) | Complement value (%) |
|---|---|---|---|---|---|
| | | GOT (K.U.) | GPT (K.U.) | | |
| 1 | Physiological saline | 120 ± 40 | 97 ± 30 | 0.09 | 100 |
| 2 | Control | 1920 ± 120 | 1030 ± 98 | 0.88 | 91.1 |
| 3 | Pionin (0.1 μg/kg) | 550 ± 160 | 510 ± 62 | 0.51 | 88.2 |
| 4 | Pionin (0.5 μg/kg) | 420 ± 30 | 300 ± 55 | 0.35 | 96.6 |
| 5 | Pionin (1 μg/kg) | 780 ± 29 | 600 ± 40 | 0.44 | 91.2 |
| 6 | Pionin (10 μg/kg) | 1036 ± 50 | 980 ± 39 | 0.77 | 87.6 |
| 7 | DSCG (i.p.) (1 mg/kg) | 400 ± 42 | 220 ± 20 | 0.29 | 88.4 |

Table 3

| No. | GalN (i.p.) | Enzyme activity GOT (K.U.) | GPT (K.U.) | Bilirubin (mg/dl) | Complement value (%) |
|---|---|---|---|---|---|
| 1 | Physiological saline | 88 ± 20 | 101 ± 31 | 0.07 | 100 |
| 2 | Control | 1330 ± 220 | 1100 ± 66 | 0.91 | 92.2 |
| 3 | Pionin (0.1 μg/kg) | 1200 ± 198 | 1003 ± 87 | 0.77 | 86.1 |
| 4 | Pionin (0.5 μg/kg) | 622 ± 100 | 550 ± 51 | 0.36 | 91.1 |
| 5 | Pionin (1 μg/kg) | 710 ± 30 | 440 ± 13 | 0.55 | 92.3 |
| 6 | Pionin (10 μg/kg) | 1350 ± 136 | 1200 ± 188 | 0.88 | 88.8 |
| 7 | DSCG (i.p.) (1 mg/kg) | 480 ± 50 | 230 ± 33 | 0.21 | 80.4 |

As is clear from the results shown in Tables 2 and 3, pionin according to the present invention can inhibit an increase in the GOT and GPT activities and in the concentration of bilirubin and, therefore, can inhibit liver disorders induced by D-galactosamine.

Example 12

Effect of methincyanine compounds on hepatic necrocytosis induced by carbon tetrachloride (in vivo)

In the evaluation tests, 6 weeks old SD male rats were used. As shown in Tables 4 to 6, 5 to 10 rats were used in each test group. The rats were freely fed or supplied with a solid feed and drinking water.

Carbon tetrachloride ($CCl_4$) was diluted with an olive oil and was peritoneally injected in each rat at a dosage of 0.5 ml/kg. To the control group, only $CCl_4$

diluted with the olive oil and a 5% gum arabic solution containing no active ingredient were administered. Furthermore, only the olive oil but no $CCl_4$ was administered to the olive oil injection group in the Tables.

The methincyanine compounds listed in Tables 4 to 6 were administered as follows. Methincyanine compounds were diluted 100 times with dextrose dissolved in 5% gum arabic solutions. All drugs were orally administered at the dosages shown in Tables 4 to 6. The administration schedules were such that the methiocyanine compounds were administered 6 times in total from 5 days before the $CCl_4$ injection to the day of the injection.

Blood was sampled from the orbital veniplex of the rat and was allowed to stand at room temperature for one hour. The blood sample was then centrifugally separated at 2800 rpm for 10 minutes. The resultant supernatant was used as a serum sample.

The determination of GOT and GPT in the serum sample was carried out by using a determination kit (available from Boeklinger-Yamanouchi Co.) and the results were recorded in an automatic analyzer (available from FLEXIGEM Co.).

The results are as shown in Tables 4 to 6.

Table 4

| Methincyanine Compounds | No. of rats | Dosage (μg/kg) | Enzyme Activity | |
|---|---|---|---|---|
| | | | GOT (U/L) | GPT (U/L) |
| Olive oil | 10 | - | $169.0 \pm 19.4^{**}$ | $48.7 \pm 5.7^{**}$ |
| Control | " | - | $1272.0 \pm 332.3$ | $346.2 \pm 85.5$ |
| Pionin | " | 1 | $678.3 \pm 76.5$ | $209.0 \pm 31.2$ |
| " | " | 3 | $754.5 \pm 108.8$ | $269.7 \pm 50.3$ |
| " | " | 10 | $559.2 \pm 56.6^{*}$ | $165.8 \pm 25.9$ |
| " | " | 30 | $946.8 \pm 110.6$ | $311.4 \pm 49.4$ |

*: $P < 0.05$ (i.e., level of significance < 5%)
**: $P < 0.01$ (i.e., level of significance < 1%)

As is clear from the results of Table 4, pionin has an activity capable of inhibiting liver necrocytosis induced by $CCl_4$, since it inhibits the increase in the GOT and GPT activities induced by $CCl_4$ also in the in vivo tests.

Table 5

| Methincyanine Compounds | No. of rats | Dosage (μg/kg) | Enzyme Activity | |
|---|---|---|---|---|
| | | | GOT (U/L) | GPT (U/L) |
| Olive oil | 5 | - | 110 ± 17** | 34 ± 4* |
| Control | 6 | - | 547 ± 83 | 173 ± 42 |
| Compound of Example 3 | 8 | 10 | 435 ± 67 | 107 ± 18 |
| Compound of Example 8 | 7 | " | 310 ± 68 | 99 ± 27 |
| Compound of Example 4 | 8 | " | 386 ± 50 | 118 ± 21 |
| Compound of Example 5 | 8 | " | 393 ± 59 | 135 ± 29 |

*: $P < 0.05$
**: $P < 0.01$

As is clear from the results shown in Table 5, the methiocyanine compounds according to the present invention having the general formula (II) (i.e., the compounds having the different substituents in $R_1$ and $R_2$) are capable of strongly inhibiting liver necrocytosis induced by $CCl_4$.

Table 6

| Methincyanine Compounds | No. of rats | Dosage (µg/kg) | Enzyme Activity | |
|---|---|---|---|---|
| | | | GOT (U/L) | GPT (U/L) |
| Olive oil | 5 | - | 138 ± 8 | 32 ± 2 |
| Control | 8 | - | 1120 ± 380 | 321 ± 123 |
| Pionin | " | 10 | 807 ± 172 | 251 ± 83 |
| Compound of Example 1 | " | " | 627 ± 92 | 198 ± 37 |

As is clear from the results shown in Table 6, the present compounds having the general formula (II) such as the compound of Example 1, i.e., $R_1$ = butyl, $R_2$ = dodecyl in general formula (II) exhibit the strong inhibiting activities against liver necrocytosis induced by $CCl_4$ when compared with pionin (i.e., $R_1 = R_2$ = heptyl).

Example 13

Preparation of tablets

The following ingredients were mixed and the mixture was formed into tablets in a conventional manner.

| Ingredient | g |
|---|---|
| Pionin | 0.2 |
| Polyvinylpyrrolidone (M.W. = 40,000 - 50,000) | 300 |
| Carboxymethyl cellulose | 190 |
| Magnesium stearate | 10 |

Thus, the desired tablets each containing 200 µg of pionin were prepared.

Example 14

Preparation of ointments

A 25 g amount of white vaseline, 22 g of stearyl alcohol, 12 g of propylene glycol, 1.5 g of sodium

lauryl sulfate, 0.025 g of ethyl p-hydroxybenzoate, and 0.013 g of propyl p-hydroxybenzoate were added to purified water to a total weight of 100 g. Thus, a hydrophilic ointment was prepared. To 100 g of the ointment thus-obtained, 50 mg of pionin was added and thoroughly mixed therewith.

Thus, the desired ointment containing 500 μg of pionin per 1 g of the ointment was obtained.

CLAIMS

1. The use of a methincyanine compound having the general formula (I) for the prophylaxis or pharmacotherapy of liver disorders of warm-blooded animals comprising administering thereto an effective amount of the methincyanine compound.

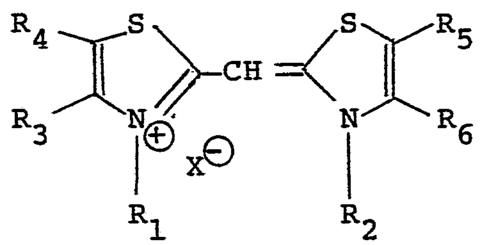

... [I]

wherein $R_1$ and $R_2$ may be the same or different and represent an unsubstituted or substituted alkyl group, $R_3$ , $R_4$ , $R_5$ , and $R_6$ independently represent a hydrogen atom or an alkyl group, and $X^{\ominus}$ represents an anion.

2. The use of the methincyanine compound as claimed in claim 1, wherein the $R_1$ and $R_2$ groups in the general formula (I) are the same alkyl or substituted alkyl groups.

3. The use of the methincyanine compound as claimed in claim 1, wherein the $R_1$ and $R_2$ groups in the general formula (I) are the different alkyl or substituted alkyl groups.

4. The use of the methincyanine compound as claimed in claim 1, wherein the effective amount of the methincyanine compound is 50 μg to 5 mg/head/day.

5. The use of the methincyanine compound as claimed in claim 1, wherein the warm-blooded animals are human beings.

6. The use of the methincyanine compound as claimed in claim 1, wherein the liver disorder or hepatic disease is toxic hepatopathy, jecur adiposum, acute hepatitis, chronic hepatitis, or hepatocirrhosis.

7. The use of a methincyanine compound having the general formula (I) defined in claim 1 for the production of a pharmaceutical preparation for the prophylaxis or

pharmacotherapy of liver disorders of warm-blooded animals.

8. The use of a methincyanine compound as claimed in claim 7, wherein the pharmaceutical preparation is in the form of a orally, parenterally, or percutaneously administration.

9. A methincyanine compound having the general formula (II):

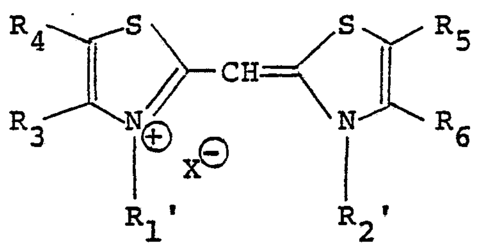

... [II]

wherein $R_1$' and $R_2$' are different from each other and represent an unsubstituted or substituted alkyl group, $R_3$ , $R_4$ , $R_5$ , and $R_6$ independently represent a hydrogen atom or an alkyl group, and $X^{\ominus}$ represents an anion.

10. A pharmaceutical composition for the prophylaxis or pharmacotherapy of liver disorders containing, as an effective component, the methincyanine compound of the general formula (II) of claim 9.

0198605

European Patent Office

**PARTIAL EUROPEAN SEARCH REPORT**
which under Rule 45 of the European Patent Convention shall be considered, for the purposes of subsequent proceedings, as the European search report

Application number
EP 86 30 1960

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A- 2 020 975 (TAKEDA)<br>* Page 1, lines 59-60 *<br>------- | 9 | C 07 D 277/22<br>A 61 K 31/425 |

TECHNICAL FIELDS SEARCHED (Int. Cl.4)

C 07 D 277/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 7-10
Claims searched incompletely:
Claims not searched: 1-6
Reason for the limitation of the search:

Method for treatment of the human or animal body by surgery or therapy (see art. 52(4) of the European Patent Convention.)

| Place of search | Date of completion of the search | Examiner |
|---|---|---|
| The Hague | 27-06-1986 | DE BUYSER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03 82